# EUROPEAN PATENT SPECIFICATION

(11) **EP 1 314 730 B1**
(45) Date of publication and mention of the grant of the patent: **10.01.2007**
(21) Application number: 01961247.2
(22) Date of filing: 31.08.2001
(51) Int. Cl.: C07D 311/82, C07D 493/08, C07D 493/10, A61B 10/00, G01N 21/76, G01N 21/78, G01N 33/483

(54) **REAGENT FOR DETERMINING SINGLET OXYGEN**
REAGENZIEN ZUR BESTIMMUNG VON ATOMAREN SAUERSTOFF
REACTIF POUR DETERMINER L'OXYGENE SINGLET

(30) Priority: 31.08.2000 JP 2000263067; 10.10.2000 JP 2000308581
(43) Date of publication of application: 28.05.2003
(73) Proprietor: DAIICHI PURE CHEMICALS CO., LTD., Chuo-ku Tokyo 103-0027 (JP); Nagano, Tetsuo, Suginami-ku, Tokyo 167-0032 (JP)
(72) Inventor: NAGANO, Tetsuo, Suginami-ku, Tokyo 167-0032 (JP); URANO, Yasuteru, Kawasaki-shi, Kanagawa 213-0013 (JP); TANAKA, Kumi, Tachikawa-shi, Tokyo 190-0002 (JP)
(74) Representative: Sternagel, Fleischer, Godemeyer & Partner
(86) International application number: PCT/JP2001/007527
(87) International publication number: WO 2002/018362

(56) References cited:
- WO-A1-99/51586
- LINDIG, B. A. ET AL.: "Determination of the lifetime of singlet oxygen in D2O using 9,10-Anthracenedipropionic acid, a water-soluble probe" J. AM. CHEM. SOC., vol. 102, no. 17, 13 August 1980 (1980-08-13), pages 5590-5593, XP002278735
- NARDELLO V ET AL: "Synthesis and Photooxidation of Sodium 1,3-Cyclohexadiene-1,4-Diethan oate: a New Colorless and Water-Soluble Trap of Singlet Oxygen" TETRAHEDRON, ELSEVIER SCIENCE PUBLISHERS, AMSTERDAM, NL, vol. 52, no. 6, 5 February 1996 (1996-02-05), pages 2031-2046, XP004104435 ISSN: 0040-4020
- NARDELLO V ET AL: "Synthesis and Properties of a New Cationic Water-Soluble Trap of Singlet Molecular Oxygen" TETRAHEDRON LETTERS, ELSEVIER SCIENCE PUBLISHERS, AMSTERDAM, NL, vol. 38, no. 42, 20 October 1997 (1997-10-20), pages 7361-7364, XP004111214 ISSN: 0040-4039
- SCHMITZ C. ET AL.: "A new access to the anthracene core" TETRAHEDRON, vol. 38, no. 10, 1982, pages 1425-1430, XP002278736
- MIURA T. ET AL.: "Rational design for modulating fluorescence properties of fluorescein-based probes by photoinduced electron transfer" J. AM. CHEM. SOC., vol. 125, 19 June 2003 (2003-06-19), pages 8666-8671, XP002278737
- NAOKI UMEZAWA ET AL.: 'Novel fluorescent probes for singlet oxygen' ANGEW. CHEM. INT. ED. vol. 38, no. 19, 1999, pages 2899 - 2901, XP002948511

## Description

### Technical Field

The present invention relates to a compound or a salt thereof useful as an agent for measurement of singlet oxygen. The present invention also relates to an agent for measurement of singlet oxygen comprising the aforementioned compound or a salt thereof.

### Background Art

It is known that, in living bodies and life phenomena, free radical species such as nitrogen monoxide are acting as a second messenger for signal transduction, and they exerts various physiological functions, for example, control of blood pressure in the circulatory system and the like. It has also been revealed that superoxides and hydrogen peroxide as active oxygen species also exert important physiological functions in the immune system and the like. However, importance of singlet oxygen as a physiologically active species, which has an analogous electronic structure, has little been elucidated so far.

Recently, singlet oxygen has been revealed to be a reactive species of photodynamic therapy, which is one of cancer therapies, and it has been suggested that various kinds of oxidases, peroxidases and the like are generating singlet oxygen in living bodies. Furthermore, it has also been revealed that oxygen molecules act as a sensor and have signal-like actions, and therefore, singlet oxygen is also suggested to have possible responsibility for important physiological functions in living bodies.

Among organs, skin suffers from direct contacts with outside air, and is a noticeably peculiar organ from a viewpoint of oxygen stress. It has been pointed out that, because skin is always exposed to oxygen and ultraviolet rays, skin lies in an environment in which oxidative damages likely occur due to active oxygens generated by ultraviolet rays or lipid peroxides generated thereby. It is suggested that accumulation of these oxidative damages is one of factors of skin retrogradation. Singlet oxygen is suggested to participate most frequently among active oxygens, however, no report has been made so far about direct measurement of singlet oxygen produced by skin-related cells.

Ten or more different methods are conventionally known as methods for measurement of singlet oxygen in living bodies, which include the chemiluminescence method, the electron spin resonance (ESR) method, the luminescence method and the like. However, these methods in common give only low specificity and sensitivity, and thus they are not reliable methods (as for the method for specific detection of singlet oxygen, see, Nagano, T., et al., Free radicals in Clinical Medicine, Vol. 7, pp.35-41, 1993, etc.). Therefore, it has been desired to develop a method for measurement of singlet oxygen superior in specificity and sensitivity to study the involvement of singlet oxygen in life phenomena.

The inventors of the present invention proposed compounds prepared by introduction of anthracene derivatives into fluorescein as means for measurement of singlet oxygen superior in specificity and sensitivity (International Patent Publication WO99/51586). By using these anthracene derivatives, singlet oxygen localized in particular cells or tissues can be measured based on a bioimaging technique. The anthracene derivatives are excellent in specificity and sensitivity, however, they have a problem that a fluorescent endoperoxide compound, which is generated by a reaction with singlet oxygen, is unstable against light. Further, these anthracene derivatives are highly lipid-soluble, which raises a problem that, for example, they are localized in cell membranes when loaded onto cells and hardly be distributed uniformly in cells. Therefore, it has been desired to develop an agent for measurement of singlet oxygen which can generate a light-stable fluorescent substance and can be uniformly distributed in cells.

### Disclosure of the Invention

An object of the present invention is to provide a compound useful as an agent for measurement of singlet oxygen. More specifically, an object of the present invention is to provide a compound useful as an agent for measurement of singlet oxygen which can generate a light-stable fluorescent substance and can be uniformly distributed in a cell. Another object of the present invention is to provide an agent for measurement of singlet oxygen comprising said compound and a method for measurement of singlet oxygen using said compound. In particular, it is an object of the present invention to provide an agent for accurate measurement of singlet oxygen localized in particular cells or tissues in living bodies by a bioimaging technique.

The inventors of the present invention conducted various studied to achieve the foregoing objects. As a result, they found that a substantially non-fluorescent compound represented by the following general formula (I) efficiently reacts with singlet oxygen to give an endoperoxide compound (II) having superior light stability, and that the resulting specific anthracene derivative is highly water-soluble. They also found that singlet oxygen localized in living cells or tissues can be measured with extremely high specificity and sensitivity by using a compound represented by the general formula (I) as an agent for measurement of singlet oxygen. The present invention was achieved on the basis of these findings.

The present invention thus provide compounds represented by the following general formula (I): 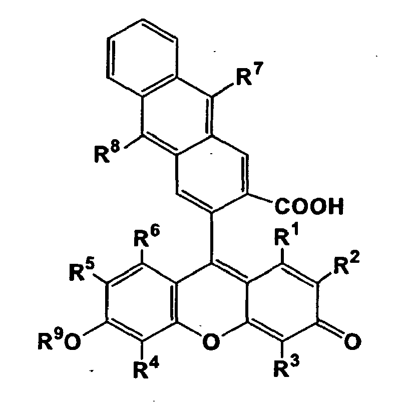 wherein R¹, R², R³, R⁴, R⁵, and R⁶ independently represent a hydrogen atom, a halogen atom, a C₁₋₆ alkyl group, or a C₁₋₆ alkoxyl group, R⁷ and R⁸ independently represent a C₁₋₄ alkyl group, and R⁹ represents a hydrogen atom, a C₁₋₁₂ alkanoyl group, or acetoxymethyl group, or salts thereof.

From another aspect of the present invention, there are also provided compounds represented by the following general formula (II): 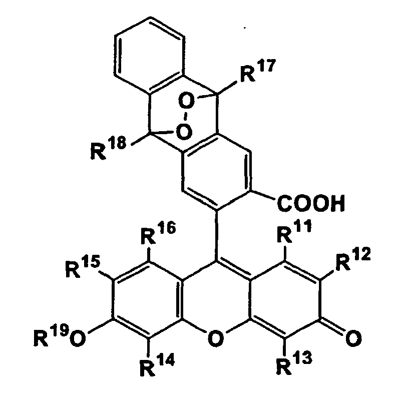 wherein R¹¹, R¹², R¹³, R¹⁴, R¹⁵, and R¹⁶ independently represent a hydrogen atom, a halogen atom, a C₁₋₆ alkyl group, or a C₁₋₆ alkoxyl group, R¹⁷ and R¹⁸ independently represent a C₁₋₄ alkyl group, and R¹⁹ represents a hydrogen atom, a C₁₋₁₂ alkanoyl group, or acetoxymethyl group, or salts thereof.

From further aspects of the present invention, there are provided agents for measurement of singlet oxygen comprising a compound represented by the aforementioned formula (I) or a salt thereof; use of the compounds represented by the aforementioned formula (I) or salts thereof for the manufacture of the aforementioned agents for measurement of singlet oxygen; and methods for measuring singlet oxygen, which comprise the steps of: (a) reacting a compound of the aforementioned formula (I) or a salt thereof with singlet oxygen, and (b) measuring fluorescence of a compound of the aforementioned formula (II) or a salt thereof produced in the above step (a).

In addition to the above, there are also provided compounds represented by the following general formula (III): 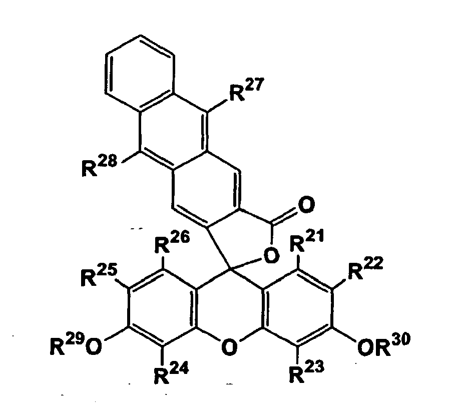 wherein R²¹, R²², R²³, R²⁴, R²⁵, and R²⁶ independently represent a hydrogen atom, a halogen atom, a C₁₋₆ alkyl group, or a C₁₋₆ alkoxyl group, R²⁷ and R²⁸ independently represent a C₁₋₄ alkyl group, and R²⁹ and R³⁰ independently represent a C₁₋₁₂ alkanoyl group or acetoxymethyl group, and
compounds represented by the following general formula (IV): 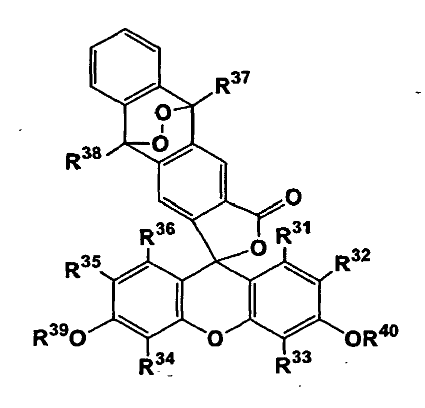 wherein R³¹, R³², R³³, R³⁴, R³⁵, and R³⁶ independently represent a hydrogen atom, a halogen atom, a C₁₋₆ alkyl group, or a C₁₋₆ alkoxyl group, R³⁷ and R³⁸ independently represent a C₁₋₄ alkyl group, and R³⁹ and R⁴⁰ independently represent a C₁₋₁₂ alkanoyl group or acetoxymethyl group. The compounds represented by the formula (III) are also useful as agents for measurement of singlet oxygen.

### Brief Explanation of the Drawings

Fig. 1 shows light stability of a compound represented by the formula (II) of the present invention (DMAX-EP) and a known compound (DPAX-1-EP).
Fig. 2 shows results of measurement of singlet oxygen using the compound of the present invention. In the figure, the arrow indicates a time when EP-1 was added. The solid line indicates the result obtained by the compound DMAX of the present invention, and the dotted line indicates the result obtained by the known compound (DPAX-1).

### Best Mode for Carrying out the Invention

The entire disclosure of Japanese Patent Application No. 2000-263067 (filed on August 31, 2000) and the entire disclosure of Japanese Patent Application No. 2000-308581 (filed on October 10, 2000) are incorporated by reference in the specification.

The terms used in this specification have the following meanings. An alkyl group or an alkyl moiety of an alkoxyl group may be linear, branched, or cyclic. More specifically, for the term of C₁₋₆ alkyl group a methyl group, ethyl group, n-propyl group, isopropyl group, cyclopropyl group, n-butyl group, sec-butyl group, tert-butyl group, cyclobutyl group, n-pentyl group, n-hexyl group, cyclohexyl group and the like may be used. As the alkyl group and the alkoxyl group, those having a linear or branched chain are preferred. As the halogen atom, although any of fluorine atom, chlorine atom, bromine atom, and iodine atom may be used, chlorine atom is preferred. The alkanoyl group may be either of linear or branched. As the alkanoyl group, for example, formyl group, acetyl group, propanoyl group and the like can be used. Acetyl group is preferred. As the C₁₋₄ alkyl group represented by R⁷, R⁸, R¹⁷, R¹⁸, R²⁷, R²⁸, R³⁷, or R³⁸, methyl group and ethyl group are preferred, and methyl group is particularly preferred.

In the formula (I), it is preferred that R¹, R², R³, R⁴, R⁵, and R⁶ are hydrogen atoms, or R¹, R³, R⁴, and R⁶ are hydrogen atoms, and R² and R⁵ are chlorine atoms. It is preferred that R⁷ and R⁸ are methyl groups, and R⁹ is a hydrogen atom, an acetyl group, or acetoxymethyl group.

In the formula (II), it is preferred that R¹¹, R¹², R¹³, R¹⁴, R¹⁵, and R¹⁶ are hydrogen atoms, or R¹¹, R¹³, R¹⁴, and R¹⁶ are hydrogen atoms, and R¹² and R¹⁵ are chlorine atoms. It is preferred that R¹⁷ and R¹⁸ are methyl groups, and R¹⁹ is a hydrogen atom, an acetyl group, or acetoxymethyl group.

In the formula (III), it is preferred that R²¹, R²², R²³, R²⁴, R²⁵, and R²⁶ are hydrogen atoms, or R²¹, R²³, R²⁴, and R²⁶ are hydrogen atoms, and R²² and R²⁵ are chlorine atoms. It is preferred that R²⁷ and R²⁸ are methyl groups, and R²⁹ and R³⁰ are both acetyl groups or acetoxymethyl groups.

In the formula (IV), it is preferred that R³¹, R³², R³³, R³⁴, R³⁵, and R³⁶ are hydrogen atoms, or R³¹, R³³, R³⁴, and R³⁶ are hydrogen atoms, and R³² and R³⁵ are chlorine atoms. It is preferred that R³⁷ and R³⁸ are methyl groups, and R³⁹ and R⁴⁰ are both acetyl groups or acetoxymethyl groups.

Among the compounds of the present invention, preferred compounds include:
(1) a compound wherein R¹, R², R³, R⁴, R⁵, and R⁶ are hydrogen atoms, R⁷ and R⁸ are methyl groups, and R⁹ is a hydrogen atom;
(2) a compound wherein R¹, R², R³, R⁴, R⁵, and R⁶ are hydrogen atoms, R⁷ and R⁸ are methyl groups, and R⁹ is an acetyl group;
(3) a compound wherein R¹, R², R³, R⁴, R⁵, and R⁶ are hydrogen atoms, R⁷ and R⁸ are methyl groups, and R⁹ is acetoxymethyl group;
(4) a compound wherein R¹, R³, R⁴, and R⁶ are hydrogen atoms, R² and R⁵ are chlorine atoms, R⁷ and R⁸ are methyl groups, and R⁹ is a hydrogen atom;
(5) a compound wherein R¹, R³, R⁴, and R⁶ are hydrogen atoms, R² and R⁵ are chlorine atoms, R⁷ and R⁸ are methyl groups, and R⁹ is an acetyl group;
(6) a compound wherein R¹, R³, R⁴, and R⁶ are hydrogen atoms, R² and R⁵ are chlorine atoms, R⁷ and R⁸ are methyl groups, and R⁹ is acetoxymethyl group;
(7) a compound wherein R¹¹, R¹², R¹³, R¹⁴, R¹⁵, and R¹⁶ are hydrogen atoms, R¹⁷ and R¹⁸ are methyl groups, and R¹⁹ is a hydrogen atom;
(8) a compound wherein R¹¹, R¹², R¹³, R¹⁴, R¹⁵, and R¹⁶ are hydrogen atoms, R¹⁷ and R¹⁸ are methyl groups, and R¹⁹ is an acetyl group;
(9) a compound wherein R¹¹, R¹², R¹³, R¹⁴, R¹⁵, and R¹⁶ are hydrogen atoms, R¹⁷ and R¹⁸ are methyl groups, and R¹⁹ is acetoxymethyl group;
(10) a compound wherein R¹¹, R¹³, R¹⁴, and R¹⁶ are a hydrogen atom, R¹² and R¹⁵ are a chlorine atom, R¹⁷ and R¹⁸ are methyl group, and R¹⁹ is a hydrogen atom;
(11) a compound wherein R¹¹, R¹³, R¹⁴, and R¹⁶ are hydrogen atoms, R¹² and R¹⁵ are chlorine atoms, R¹⁷ and R¹⁸ are methyl groups, and R¹⁹ is an acetyl group;
(12) a compound wherein R¹¹, R¹³, R¹⁴, and R¹⁶ are hydrogen atoms, R¹² and R¹⁵ are chlorine atoms, R¹⁷ and R¹⁸ are methyl groups, and R¹⁹ is acetoxymethyl group;
(13) a compound wherein R²¹, R²², R²³, R²⁴, R²⁵, and R²⁶ are hydrogen atoms, R²⁷ and R²⁸ are methyl groups, and R²⁹ and R³⁰ are acetyl groups;
(14) a compound wherein R²¹, R²², R²³, R²⁴, R²⁵, and R²⁶ are hydrogen atoms, R²⁷ and R²⁸ are methyl groups, and R²⁹ and R³⁰ are acetoxymethyl groups;
(15) a compound wherein R²¹, R²³, R²⁴, and R²⁶ are hydrogen atoms, R²² and R²⁵ are chlorine atoms, R²⁷ and R²⁸ are methyl groups, and R²⁹ and R³⁰ are acetyl groups;
(16) a compound wherein R²¹, R²³, R²⁴, and R²⁶ are hydrogen atoms, R²² and R²⁵ are chlorine atoms, R²⁷ and R²⁸ are methyl groups, and R²⁹ and R³⁰ are acetoxymethyl groups;
(17) a compound wherein R³¹, R³², R³³, R³⁴, R³⁵, and R³⁶ are hydrogen atoms, R³⁷ and R³⁸ are methyl groups, and R³⁹ and R⁴⁰ are acetyl groups;
(18) a compound wherein R³¹, R³², R³³, R³⁴, R³⁵, and R³⁶ are hydrogen atoms, R³⁷ and R³⁸ are methyl groups, and R³⁹ and R⁴⁰ are acetoxymethyl groups;
(19) a compound wherein R³¹, R³³, R³⁴, and R³⁶ are hydrogen atoms, R³² and R³⁵ are chlorine atoms, R³⁷ and R³⁸ are methyl groups, and R³⁹ and R⁴⁰ are acetyl groups; and
(20) a compound wherein R³¹, R³³, R³⁴, and R³⁶ are hydrogen atoms, R³² and R³⁵ are chlorine atoms, R³⁷ and R³⁸ are methyl groups, and R³⁹ and R⁴⁰ are acetoxymethyl groups. Among them, a particularly preferred compound is the aforementioned compound (1).

The compounds of the formula (I) and the formula (II) can exist as a base addition salt. Examples of the base addition salts include, for example, metal salts such as sodium salts, potassium salts, calcium salts, and magnesium salt, ammonium salts, organic amine salts such as triethylamine salts, piperidine salts and morpholine salts and the like. However, salts of the compounds of the present invention are not limited to these examples. Among them, physiologically acceptable water-soluble base addition salts can suitably be used for the agent and method for measurement of the present invention. Further, the compounds of the formula (I) and the formula (II) in free forms or salts thereof may exist as hydrates or solvates, and any of these substances fall within the scope of the present invention. The types of solvents that form the solvates are not particularly limited. For example, solvents such as ethanol, acetone and isopropanol can be exemplified.

The compounds of the formula (I) and the formula (II) may have one or more asymmetric carbons depending on the type of the substituent(s), and optical isomers or diastereoisomers may exist. Further, depending on the type of R¹ and/or R⁶, or R¹¹ and/or R¹⁶, optical isomers due to rotation hindrance may exist. These isomers in pure forms, any mixtures of these isomers, racemates and the like fall within the scope of the present invention. In addition, the compounds of the formula (I) and the formula (II) of the present invention may form a lactone ring and exist as compounds having a structure corresponding to the fundamental structure of the compounds of the formula (III) or the formula (IV), or they may also exist as other tautomers. It should be understood that these compounds in which the lactone ring is formed and other isomers fall within the scope of the present invention. Optically active isomers based on the aforementioned lactone formation also fall within the scope of the present invention.

Methods for preparing the compounds of the present invention are not particularly limited. For example, the compounds of the present invention can be prepared by the method described in International Patent Publication WO99/51586. Further, the method for preparing the compounds of the present invention will be described more specifically and in more detail in examples of the specification. Therefore, those skilled in the art can prepare any of the compounds of the present invention by referring to the explanations of the manufacturing method mentioned in the above schemes and specific explanations in the examples, and by appropriately choosing starting materials and agents, and by suitably altering or modifying reaction conditions, reaction steps and the like as required. A target compound can sometimes be efficiently prepared by performing the reaction after protection of a certain functional group as required in the reaction steps. Detailed explanations of protective groups are given in, for example, Protective Groups in Organic Synthesis, T.W. Greene, John Wiley & Sons, Inc., 1981 and the like, and those skilled in the art can choose suitable protective groups.

In the above preparations, isolation and purification of the products can be performed by a suitable combination of techniques used in ordinary organic synthesis, for example, filtration, extraction, washing, dehydration, concentration, crystallization, various chromatography techniques and the like. The synthetic intermediates in the above steps can be used for the subsequent reactions without particular purification. Where preparation of a salt of the compound of the present invention is desired, when a salt of each compound is obtained in the above preparation, the resulting salt, per se, may be purified. When a compound in a free form is obtained, the compound in a free form can be dissolved or suspended in a suitable solvent and added with a base to form a salt, which may be purified as required.

The compounds represented by the aforementioned formula (I) and salts thereof have a property of reacting with singlet oxygen under a mild condition, for example, a physiological condition, to give a corresponding compound of the aforementioned formula (II) or a salt thereof. The compounds of the formula (I) and salts thereof are substantially non-fluorescent, whereas the compounds of the formula (II) and salts thereof have a property of emitting fluorescence with a high intensity. Therefore, by subjecting a compound of the aforementioned formula (I) or a salt thereof to reaction with singlet oxygen, and then measuring fluorescence of a produced compound of the aforementioned formula (II) or a salt thereof, singlet oxygen can be measured. The compounds of the formula (I) or salts thereof have a property that they do not substantially react with oxygen radicals and the like, but specifically react with singlet oxygen. Further, the compounds of the formula (II) and salts thereof have extremely high fluorescence intensity. Therefore, by using the compound of the formula (I) or a salt thereof as an agent for measurement of singlet oxygen, singlet oxygen localized in individual cells or in particular tissues can be accurately measured. The compounds of the formula (I) or salts thereof have a excellent property of being uniformly distributed in a cell without being localized in the cell membrane. Further, the compounds of the formula (II) and salts thereof generated by the reaction with singlet oxygen have sensitivity higher than the anthracene derivative described in International Patent Publication WO99/51586.

The term "measurement" used in the present specification should be construed in its broadest sense, including measurements performed for the purpose of quantification, qualification, diagnosis or the like, as well as tests or detections and the like. The method for measurement of singlet oxygen of the present invention generally comprises the steps of (a) reacting a compound of the aforementioned formula (I) or a salt thereof with singlet oxygen, and (b) measuring fluorescence of a compound of the aforementioned formula (II) or a salt thereof produced in the above step (a). The fluorescence of the compound of the aforementioned formula (II) or a salt thereof may be measured by an ordinary method. A method of measuring fluorescence spectrum in vitro, a method of measuring fluorescence spectrum in vivo by using a bioimaging technique and the like may be employed. For example, when quantification is desired, it is preferred to prepare a calibration curve beforehand according to a conventional method. As a quantitative singlet oxygen generation system, for example, the naphthalene endoperoxide system (Saito, I, .et al., J. Am. Chem. Soc., 107, pp.6329-6334, 1985) and the like can be used.

A compound of the formula (I) wherein R⁹ is a C₁₋₁₂ alkanoyl group or acetoxymethyl group or a salt thereof, or a compound of the formula (III), after it passes through a cell membrane and is taken up into a cell, in which the alkanoyl group or acetoxymethyl group is hydrolyzed by an enzyme such as an intracellular esterase gives a hydrolyzed product [a compound of the formula (I) wherein R⁹ is a hydrogen atom or a salt thereof]. The resulting hydrolyzed product reacts with singlet oxygen in the cell without being easily excreted extracellularly to give a compound of the formula (II) wherein R¹⁹ is a hydrogen atom. Therefore, if these compounds are used as agents for the measurement, singlet oxygen localized in individual cells can be measured by a bioimaging technique with high sensitivity.

As the agent for measurement of singlet oxygen of the present invention, a compound of the formula (I) or a salt thereof or a compound of the aforementioned formula (III) per se may be used. They may also be used as a composition formulated with additives ordinarily used for preparation of agents, if desired. For example, as additives for use of the agent in a physiological condition, additives such as dissolving aids, pH modifiers, buffers, isotonic agents and the like can be used, and amounts of these additives can suitably be chosen by those skilled in the art. The compositions may be provided as compositions in appropriate forms, for example, powdery mixtures, lyophilized products, granules, tablets, solutions and the like. Since the method for measurement of singlet oxygen is specifically disclosed in International Patent Publication WO99/51586, those skilled in the art can use the agents for measurement of singlet oxygen of the present invention by referring to the aforementioned publication. The disclosure of International Patent Publication WO99/51586 is herein incorporated by reference.

### Examples

The present invention will be more specifically explained with reference to the following examples. However, the scope of the present invention is not limited to the following examples.

### Example 1: Preparation of compounds of the present invention

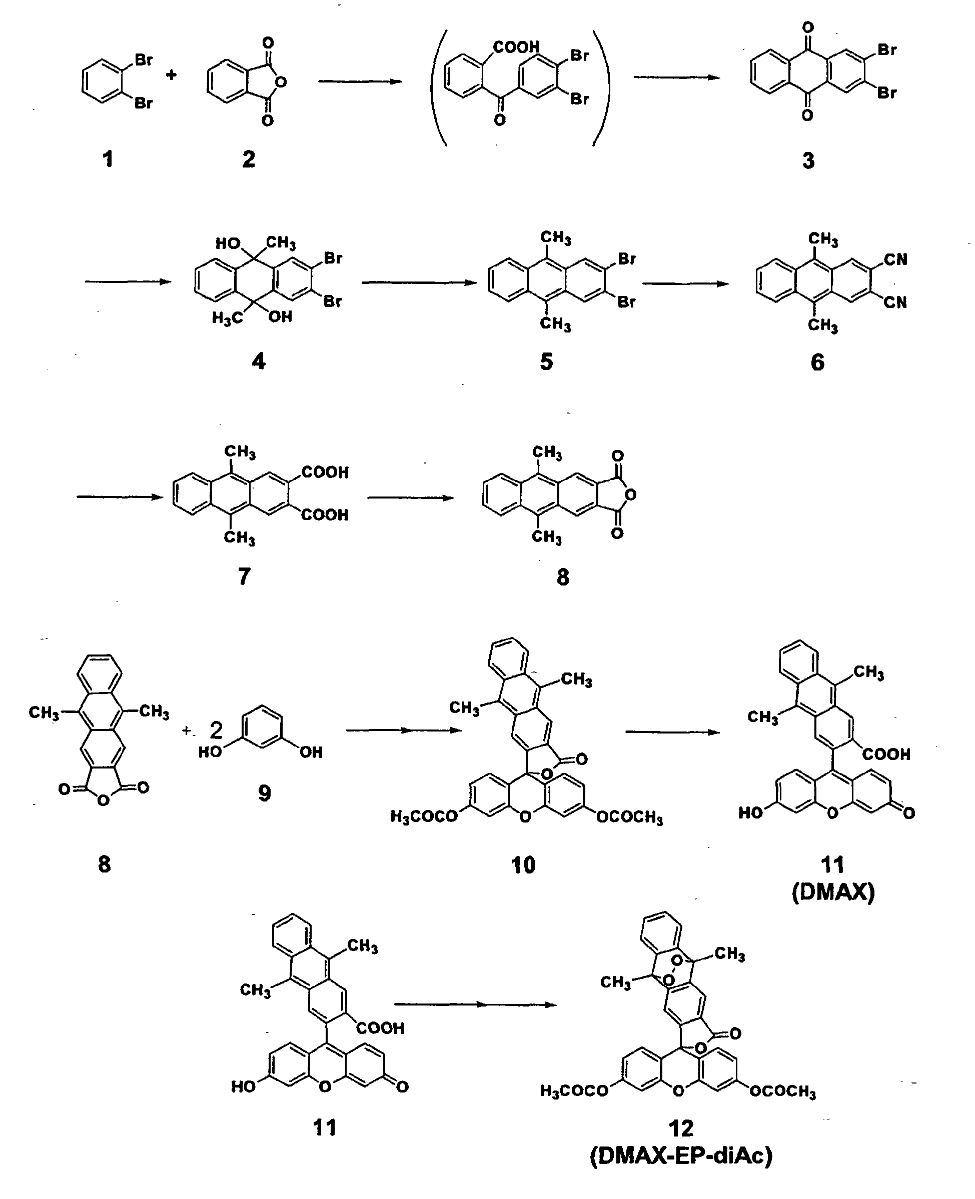

### (1) Synthesis of 2,3-dibromoanthraquinone 3

1,2-Dibromobenzene 1 (6 ml) was added with pulverized phthalic anhydride 2 (2.24 g, 15.1 mmol) and aluminum chloride (III) (4.4 g, 33.0 mmol), and heated at 150°C for 1 hour. The cooled reaction mixture was added with 2 N HCl, and extracted with benzene. The benzene layer was extracted with 2 M aqueous sodium hydroxide, and the aqueous layer was washed with ether, adjusted to about pH 2.5 with 6 M HCl, and extracted with ether. The organic layer was washed with saturated brine and dried over sodium sulfate, and ether was evaporated under reduced pressure. The resulting solid was dissolved in concentrated sulfuric acid (20 mL) and used without treatment in the subsequent reaction. The reaction mixture was gradually heated to 125°C over 1 hour and then maintained at 125°C for 30 minutes. The reaction mixture was cooled and poured into ice, and the precipitates were collected by filtration. The precipitates were dried and purified by silica gel chromatography (solvent: CH₂Cl₂/n-hexane = 2/1) to obtain Compound 3 (1.38 g, yield: 25%, light yellow powder).
¹H NMR (CDCl₃): δ 7.84 (dd, 2H, J=3.1, 5.7 Hz), 8.32 (dd, 2H, J=3.1, 5.7 Hz), 8.53 (s, 2H)
MS (EI⁺): 364:366:368 = 1:2:1 (M⁺)
m.p.: > 300°C

### (2) Synthesis of 2,3-dibromo-9,10-dimethyl-9,10-dihydroanthracene 4

Compound 3 (1.22 g, 3.33 mmol) was dissolved in distilled THF (150 ml). The solution was gradually added with methylmagnesium chloride (3 M in THF, 4.5 ml) under argon atmosphere and refluxed with heating for 4 hours. The cooled reaction mixture was treated with aqueous saturated ammonium chloride, and THF was evaporated under reduced pressure. The remaining reaction mixture was extracted with methylene chloride, and the organic layer was washed with saturated brine and dried over sodium sulfate. The methylene chloride was evaporated under reduced pressure to obtain crude product 4. The crude product was purified by silica gel chromatography (solvent: CH₂Cl₂) to obtain Compound 4 (995 mg, yield: 75%, light yellow powder).
¹H NMR (CDCl₃): cis: δ 1.63 (s, 6H), 7.41-7.45 (m, 2H), 7.79-7.83 (m, 2H), 8.11 (s, 2H), trans: δ 1.86 (s, 6H), 7.41-7.45 (m, 2H), 7.79-7.83 (m, 2H), 8.01 (s, 2H)
MS (EI⁺): 396:398:400 = 1:2:1 (M⁺)

### (3) Synthesis of 2,3-dibromo-9,10-dimethylanthracene 5

Compound 4 (1.08 g, 2.71 mmol) was dissolved in acetic acid (28 ml), added with tin(II) chloride dihydrate (12.8 g) and concentrated hydrochloric acid (12 ml) and refluxed with heating under argon atmosphere for 1 hour. The cooled reaction mixture was poured into water (500 ml), and the precipitates were collected by filtration. The precipitates were dried and purified by silica gel chromatography (solvent: CH₂Cl₂/hexane = 1/2) to obtain Compound 5 (744 mg, yield: 78%, yellow powder).
¹H NMR (CDCl₃): δ 3.04 (s, 6H), 7.55 (dd, 2H, J=3.5, 7.0 Hz), 8.30 (dd, 2H, J=3.5, 7.0 Hz), 8.62 (s, 2H)
MS (EI⁺): 362:364:366 = 1:2:1 (M⁺)

### (4) Synthesis of 9,10-dimethylanthracene-2,3-dicarbonitrile 6

Compound 5 (730 mg, 2.00 mmol) was dissolved in distilled DMF (45 ml), added with copper(I) cyanide (694 mg, 7.74 mmol), and refluxed with heating under argon atmosphere for 9 hours. The reaction mixture was cooled and then added with 12.5% aqueous ammonia (90 ml), and the precipitates were collected by filtration. The precipitates were dried and then purified by silica gel chromatography (solvent: CH₂Cl₂/hexane = 2/5) to obtain Compound 6 (255 mg, yield: 50%, yellow crystals). ¹H NMR (CDCl₃): δ 3.14 (s, 6H), 7.73 (dd, 2H, J=3.2, 6.8 Hz), 8.41 (dd, 2H, J=3.2, 6.8 Hz), 8.86 (s, 2H)
MS (EI⁺): 256 (M⁺)
m.p.: 266°C (decomp.)

### (5) Synthesis of 9,10-dimethyl-2,3-anthracenedicarboxylic acid 7

Compound 6 (330 mg, 1.29 mmol) was dissolved in 3 M butanolic potassium hydroxide (50 ml) and refluxed with heating under argon atmosphere for 10 hours. The cooled reaction mixture was treated with 2 N HCl and extracted with ether. The organic layer was washed with saturated brine and dried over sodium sulfate. The ether was evaporated under reduced pressure, and the residue was purified by silica gel chromatography (solvent: CH₂Cl₂/methanol = 20/1) to obtain Compound 7 (268 mg, yield: 71%, yellow powder).
¹H NMR (DMSO-d₆): δ 3.08 (s, 6H), 7.66 (dd, 2H, J=1.6, 6.8 Hz), 8.43 (dd, 2H, J=1.6, 6.8 Hz), 8.66 (s, 2H)
MS (EI⁺): 294 (M⁺)

### (6) Synthesis of 9,10-dimethyl-2,3-anthracenedicarboxylic anhydride 8

Compound 7 (645 mg, 2.19 mmol) was added with acetic anhydride (110 ml) and refluxed with heating for 10 minutes. The reaction mixture was cooled to precipitate crystals, and the precipitates were collected by filtration to obtain Compound 8 (367 mg, yield: 61%, red crystals).
¹H NMR (CDCl₃): δ 3.23 (s, 6H), 7.73 (dd, 2H, J=1.5, 7.0 Hz), 8.44 (dd, 2H, J=1.5, 7.0 Hz), 9.12 (s, 2H)
MS (EI⁺): 276 (M⁺)
m.p.: 278°C

### (7) Synthesis of DMAX-diAc 10

Resorcinol 9 (681 mg, 6.18 mmol) was dissolved in methanesulfonic acid (6.6 ml) and added with Compound 8 (367 mg, 1.33 mmol). The reaction mixture was heated at 85°C with light shielding under argon atmosphere for 24 hours. The cooled reaction mixture was poured into ice water (43 ml), and the precipitates were collected by filtration and dried. The resulting solid was dissolved in acetic anhydride (8 ml), added with pyridine (4 ml), and stirred at room temperature under argon atmosphere for 10 minutes. The reaction mixture was poured into 2% hydrochloric acid at 0°C and extracted with methylene chloride. The organic layer was washed with saturated brine and dried over sodium sulfate. The methylene chloride was evaporated under reduced pressure, and the precipitates were purified by silica gel chromatography (solvent: CH₂Cl₂). The product was recrystallized from benzene to obtain Compound 10 (294 mg, yield: 48%, yellow crystals).
¹H NMR (CDCl₃): δ 2.31 (s, 6H), 2.99 (s, 3H), 3.29 (s, 3H), 6.79 (dd, 2H, J=8.7, 2.2 Hz), 6.92 (d, 2H, J=8.7 Hz), 7.14 (d, 2H, J=2.2 Hz), 7.59-7.62 (m, 2H), 8.11 (s, 1H), 8.30-8.42 (m, 2H), 9.20 (s, 1H)
MS (FAB⁺): 461 (M⁺+1)
m.p.: 280°C (decomp.)

### (8) Synthesis of DMAX 11

Compound 10 (30 mg, 65.1 µ mol) was dissolved in THF (5 ml), methanol (5 ml), and distilled water (0.8 ml). This solution was added with commercially available aqueous ammonia (25-28%, 1.4 ml), and stirred at room temperature for 5 minutes. Then, the reaction mixture was filtered and diluted with distilled water (60 ml). The reaction mixture was adjusted to pH 2 with 10% HCl, and THF and methanol were evaporated under reduced pressure. The remaining reaction mixture was extracted with ether, washed with saturated brine, and dried over magnesium sulfate. The ether was evaporated under reduced pressure to obtain Compound 11 (18 mg, yield: 60%, reddish brown powder).
¹H NMR (DMSO-d₆): δ 3.15 (s, 3H), 3.17 (s, 3H), 6.49-6.52 (m, 2H), 6.65-6.69 (m, 4H), 7.63-7.67 (m, 2H), 8.14 (s, 1H), 8.35-8.50 (m, 2H), 9.09 (s, 1H)
MS (FAB⁺): 461 (M⁺+1)
m.p.: 236°C (decomp)

### (9) Synthesis of DMAX-EP-diAc 12

A solution of Compound 11 (104 mg, 0.23 mmol) dissolved in dimethyl sulfoxide (DMSO, 20 ml) was added to Buffer A (180 ml, Buffer A: 9.3 mM sodium hydroxide, 4.8 mM sodium hydrogencarbonate, 9.4 mM sodium carbonate, and 138 mM sodium molybdate dihydrate). This reaction mixture was added with 30% aqueous hydrogen peroxide twice (5 ml each) with an interval of 15 minutes. The mixture was appropriately cooled so that the reaction temperature was not undesirably increased and maintained around 20°C as near as possible. The reaction mixture was made acidic with phosphoric acid and then extracted with ether. The organic layer was washed with saturated brine and dried over magnesium sulfate, and the ether was evaporated under reduced pressure. The resulting solid was added with acetic anhydride and pyridine and stirred at room temperature for 10 minutes. The reaction mixture was poured into 2% hydrochloric acid at 0°C and extracted with methylene chloride. The organic layer was washed with saturated brine and dried over sodium sulfate. The methylene chloride was evaporated under reduced pressure, and the residue was purified by silica gel chromatography (solvent: CH₂Cl₂) to obtain Compound 12 (59 mg, yield: 45%).
¹H NMR (CDCl₃): δ 2.06 (s, 3H), 2.26 (s, 3H), 2.28 (s, 3H), 2.33 (s, 3H), 6.67-6.74 (m, 2H), 6.84-6.99 (m, 2H), 7.06-7.13 (m, 2H), 7.14 (s, 1H), 7.30-7.49 (m, 4H), 8.01 (s, 1H)
MS (FAB⁺): 577 (M⁺+1)

### Example 2: Light stability of DMAX-EP

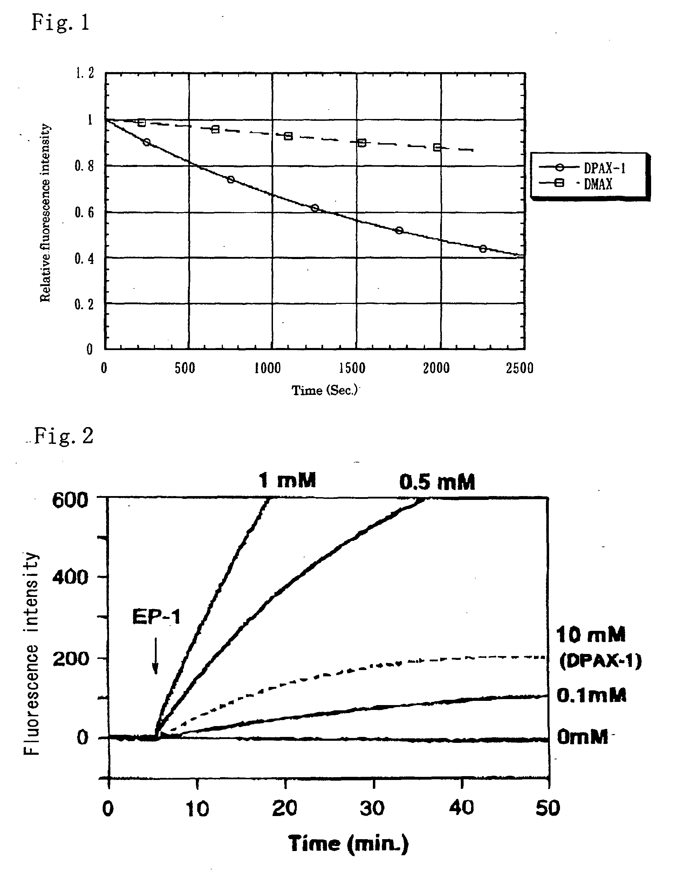

5.0 µ M aqueous solutions of DMAX-EP (endoperoxide of Compound 11 in the above scheme) and DPAX-1-EP (endoperoxide of Compound 13 described in International Patent Publication WO99/51586, Example 1) [0.1 M phosphate buffer (pH 7.4) containing 0.1% DMSO as a cosolvent] were each placed in a fluorescent cell and exposed to an excitation light of 491 nm at 37°C with stirring, and fluorescence was measured at 520 nm by using a fluorometer while the solution was continuously exposed to the excitation light. The results are shown in Fig. 1. Photobleaching of DMAX-EP was 5.4 times slower than that of DPAX-1-EP, which verified excellent light stability.

### Example 3: Measurement of singlet oxygen

Singlet oxygen was generated continuously with time under a neutral condition at 37°C in given amounts by using a naphthalene endoperoxide compound EP-1 (Saito, I, .et al., J. Am. Chem. Soc., 107, pp.6329-6334, 1985) as a singlet oxygen generation system. Fluorescence was measured in the presence of DMAX and DPAX-1 [100 mM phosphate buffer (pH 7.4), DMSO (0.1%)]. The results are shown in Fig. 2. In the presence of DMAX, an increase of fluorescence intensity dependent on the generated amount of singlet oxygen was observed as the concentration of EP-1 was increased from 0.1 mM to 0.5 mM and to 1.0 mM (The results were shown by the solid line in Fig. 2. EP-1 at each concentration was added into the system at each of the times indicated by arrows. Numerical values indicate EP-1 concentrations). Further, the increase of fluorescence intensity obtained by the addition of 0.5 mM EP-1 in the presence of DMAX was far larger than that obtained by the addition of 10 mM EP-1 in the presence of DPAX-1 (dotted line in the figure), which revealed that DMAX had much higher sensitivity than that of DPAX-1.

### Industrial Applicability

The compounds of the present invention are useful as agents for measurement of singlet oxygen. The compounds have much higher sensitivity compared with conventional agents for the measurement with a similar structure, and have significantly suppressed photobleaching of fluorescent compounds to be measured. Therefore, the agents for measurement of singlet oxygen of the present invention are extremely useful as agents for accurately measuring singlet oxygen in biological samples.

## Claims

1. A compound represented by the following general formula (I): 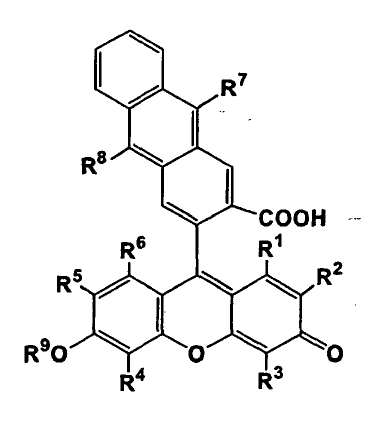 wherein R¹, R², R³, R⁴, R⁵, and R⁶ independently represent a hydrogen atom, a halogen atom, a C₁₋₆ alkyl group, or a C₁₋₆ alkoxyl group, R⁷ and R⁸ independently represent a C₁₋₄ alkyl group, and R⁹ represents a hydrogen atom, a C₁₋₁₂ alkanoyl group, or acetoxymethyl group, or a salt thereof.

2. The compound or a salt thereof according to claim 1, wherein R¹, R², R³_{,} R⁴_{,} R⁵, and R⁶ represent a hydrogen atom, R⁷ and R⁸ represent methyl group, and R⁹ represents a hydrogen atom.

3. The compound or a salt thereof according to claim 1, wherein R¹, R³, R⁴, and R⁶ represent a hydrogen atom, R² and R⁵ represent a chlorine atom, R⁷ and R⁸ represent methyl group, and R⁹ represents a hydrogen atom.

4. An agent for measurement of singlet oxygen, which comprises a compound or a salt thereof according to any one of claims 1 to 3.

5. A compound represented by the following general formula (II): 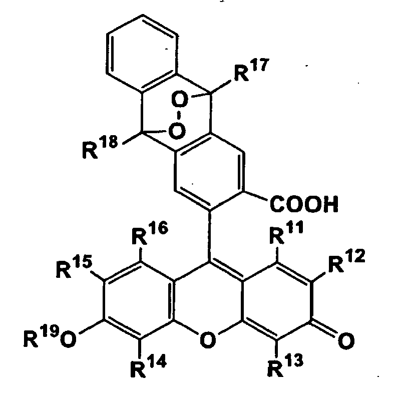 wherein R¹¹, R¹², R¹³, R¹⁴, R¹⁵, and R¹⁶ independently represent a hydrogen atom, a halogen atom, a C₁₋₆ alkyl group, or a C₁₋₆ alkoxyl group, R¹⁷ and R¹⁸ independently represent a C₁₋₄ alkyl group, and R¹⁹ represents a hydrogen atom, a C₁₋₁₂ alkanoyl group, or acetoxymethyl group, or a salt thereof.

6. A method for measurement of singlet oxygen, which comprises the steps of:
(a) reacting a compound represented by the general formula (I) or a salt thereof according to claim 1 with singlet oxygen, and
(b) measuring fluorescence of a compound represented by the general formula (II) or a salt thereof according to claim 5 produced in the above step (a).

7. A compound represented by the following general formula (III): 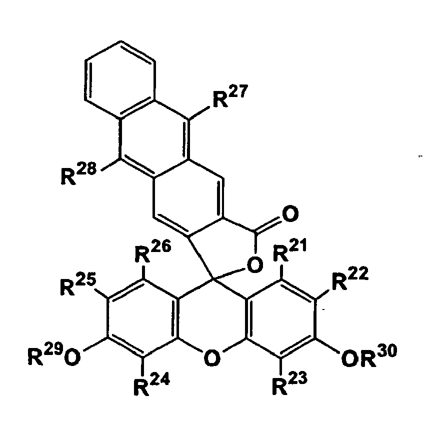 wherein R²¹, R²², R²³, R²⁴, R²⁵, and R²⁶ independently represent a hydrogen atom, a halogen atom, a C₁₋₆ alkyl group, or a C₁₋₆ alkoxyl group, R²⁷ and R²⁸ independently represent a C₁₋₄ alkyl group, and R²⁹ and R³⁰ independently represent a C₁₋₁₂ alkanoyl group or acetoxymethyl group, or a salt thereof.

8. The compound or a salt thereof according to claim 7, wherein R²¹, R²², R²³, R²⁴_{,} R²⁵, and R²⁶ represent a hydrogen atom, R²⁷ and R²⁸ represent methyl group, and R²⁹ are R³⁰ represent acetyl group.

9. The compound or a salt thereof according to claim 7, wherein R²¹, R²³, R²⁴, and R²⁶ represent a hydrogen atom, R²² and R²⁵ represent a chlorine atom, R²⁷ and R²⁸ represent methyl group, and R²⁹ and R³⁰ represent acetyl group.

10. An agent for measurement of singlet oxygen, which comprises a compound or a salt thereof according to any one of claims 7 to 9.

11. A compound represented by the following general formula (IV): 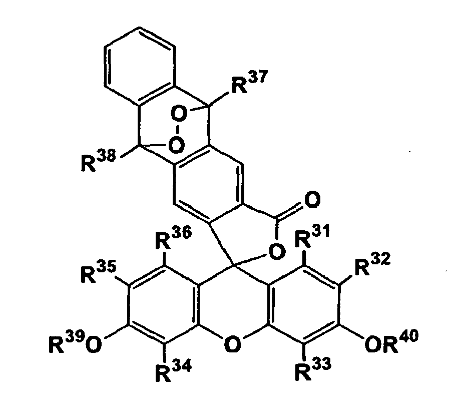 wherein R³¹, R³², R³³, R³⁴, R³⁵ and R³⁶ independently represent a hydrogen atom, a halogen atom, a C₁₋₆ alkyl group, or a C₁₋₆ alkoxyl group, R³⁷ and R³⁸ independently represent a C₁₋₄ alkyl group, and R³⁹ and R⁴⁰ independently represent a C₁₋₁₂ alkanoyl group or acetoxymethyl group, or a salt thereof.

## Patentansprüche

1. Verbindung, dargestellt durch die folgende allgemeine Formel (I): 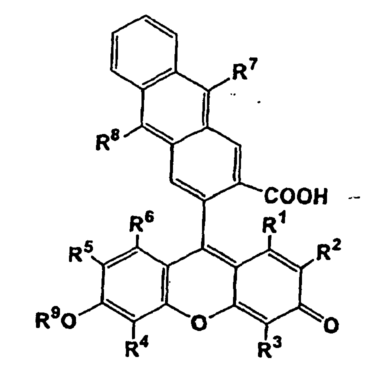 worin R¹, R², R³, R⁴, R⁵ und R⁶ unabhängig voneinander ein Wasserstoff-Atom, ein Halogen-Atom, eine C₁₋₆-Alkyl-Gruppe oder eine C₁₋₆-AlkoxyGruppe bedeuten, R⁷ und R⁸ unabhängig voneinander eine C₁₋₄-AlkylGruppe bedeuten, und R⁹ ein Wasserstoff-Atom, eine C₁₋₁₂-Alkanoyl-Gruppe oder eine Acetoxymethyl-Gruppe bedeutet, oder ein Salz derselben.

2. Verbindung oder deren Salz nach Anspruch 1, worin R¹, R², R³, R⁴, R⁵ und R⁶ ein Wasserstoff-Atom bedeuten, R⁷ und R⁸ für eine Methyl-Gruppe stehen, und R⁹ ein Wasserstoff-Atom bedeutet.

3. Verbindung oder deren Salz nach Anspruch 1, worin R¹, R³, R⁴ und R⁶ ein Wasserstoff-Atom bedeuten, R² und R⁵ ein Chlor-Atom bedeuten, R⁷ und R⁸ für eine Methyl-Gruppe stehen, und R⁹ ein Wasserstoff-Atom bedeutet.

4. Agens zur Messung von Singulett-Sauerstoff, umfassend eine Verbindung oder deren Salz nach einem der Ansprüche 1 bis 3.

5. Verbindung, dargestellt durch die folgende allgemeine Formel (II): 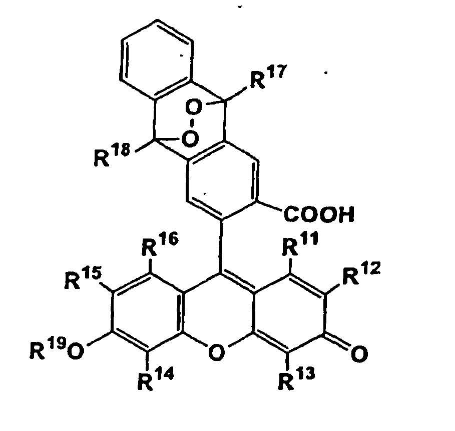 worin R¹¹, R¹², R¹³, R¹⁴, R¹⁵ und R¹⁶ unabhängig voneinander ein Wasserstoff-Atom, ein Halogen-Atom, eine C₁₋₆-Alkyl-Gruppe oder eine C₁₋₆-Alkoxy-Gruppe bedeuten, R¹⁷ und R¹⁸ unabhängig voneinander eine C₁₋₄-AlkylGruppe bedeuten, und R¹⁹ ein Wasserstoff-Atom, eine C₁₋₁₂-Alkanoyl-Gruppe oder eine Acetoxymethyl-Gruppe bedeutet, oder ein Salz derselben.

6. Verfahren zur Messung von Singulett-Sauerstoff, umfassend die Schritte:
a) Umsetzen einer durch die allgemeine Formel (I) dargestellten Verbindung oder eines Salzes derselben nach Anspruch 1 mit Singulett-Sauerstoff, und
b) Messen der Fluoreszenz einer durch die allgemeine Formel (II) dargestellten Verbindung oder eines Salzes derselben nach Anspruch 5, hergestellt im obigen Schritt a).

7. Verbindung, dargestellt durch die folgende allgemeine Formel (III): 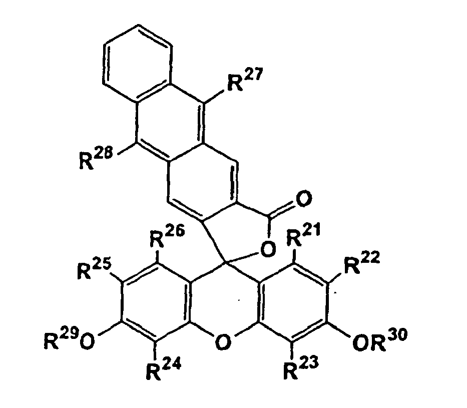 worin R²¹, R²², R²³, R²⁴, R²⁵ und R²⁶ unabhängig voneinander ein Wasserstoff-Atom, ein Halogen-Atom, eine C₁₋₆-Alkyl-Gruppe oder eine C₁₋₆-Alkoxy-Gruppe bedeuten, R²⁷ und R²⁸ unabhängig voneinander eine C₁₋₄-AlkylGruppe bedeuten, und R²⁹ und R³⁰ unabhängig voneinander eine C₁₋₁₂-Alkanoyl-Gruppe oder eine Acetoxymethyl-Gruppe bedeuten, oder ein Salz derselben.

8. Verbindung oder deren Salz nach Anspruch 7, worin R²¹, R²², R²³, R²⁴, R²⁵ und R²⁶ ein Wasserstoff-Atom bedeuten, R²⁷ und R²⁸ für eine Methyl-Gruppe stehen, und R²⁹ und R³⁰ eine Acetyl-Gruppe bedeuten.

9. Verbindung oder deren Salz nach Anspruch 7, worin R²¹, R²³, R²⁴ und R²⁶ ein Wasserstoff-Atom bedeuten, R²² und R²⁵ für ein Chlor-Atom stehen, R²⁷ und R²⁸ für eine Methyl-Gruppe stehen, und R²⁹ und R³⁰ eine Acetyl-Gruppe bedeuten.

10. Agens zur Messung von Singulott-Sauerstoff, umfassend eine Verbindung oder ein Salz derselben nach einem der Ansprüche 7 bis 9.

11. Verbindung, dargestellt durch die folgende allgemeine Formel (IV): 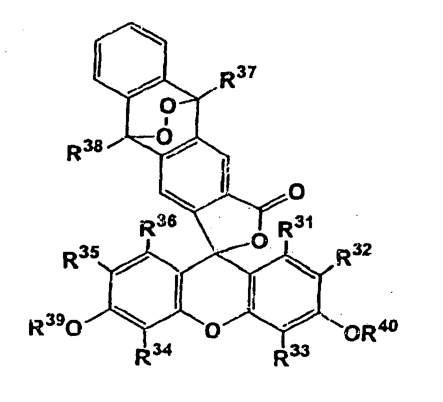 worin R³¹, R³², R³³, R³⁴, R³⁵ und R³⁶ unabhängig voneinander ein Wasserstoff-Atom, ein Halogen-Atom, eine C₁₋₆-Alkyl-Gruppe oder eine C₁₋₃-Alkoxy-Gruppe bedeuten, R³⁷ und R³⁸ unabhängig voneinander eine C₁₋₄-AlkylGruppe bedeuten, und R³⁹ und R⁴⁰ unabhängig voneinander eine C₁₋₁₂-Alkanoyl-Gruppe oder eine Acetoxymethyl-Gruppe bedeuten, oder ein Salz derselben.

## Revendications

1. Composé représenté par la formule générale suivante (I) : 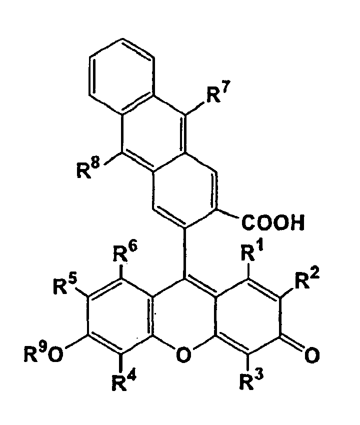 dans laquelle R¹, R², R³, R⁴, R⁵ et R⁶ représentent indépendamment un atome d'hydrogène, un atome d'halogène ou un groupe alkyle en C₁ à C₆, ou un groupe alcoxy en C₁ à C₆, R⁷ et R⁸ représentent indépendamment un groupe alkyle en C₁ à C₄, et R⁹ représente un atome d'hydrogène, un groupe alcanoyle en C₁ à C₁₂, ou un groupe acétoxyméthyle, ou un sel de celui-ci.

2. Composé ou sel de celui-ci selon la revendication 1, dans lequel R¹, R², R³, R⁴, R⁵ et R⁶ représentent un atome d'hydrogène, R⁷ et R⁸ représentent un groupe méthyle et R⁹ représente un atome d'hydrogène.

3. Composé ou sel de celui-ci selon la revendication 1, dans lequel R¹, R³, R⁴ et R⁶ représentent un atome d'hydrogène, R² et R⁵ représentent un atome de chlore, R⁷ et R⁸ représentent un groupe méthyle et R⁹ représente un atome d'hydrogène.

4. Agent pour la mesure d'un oxygène singulet, qui comprend un composé ou un sel de celui-ci selon l'une quelconque des revendications 1 à 3.

5. Composé représenté par la formule générale suivante (II) : 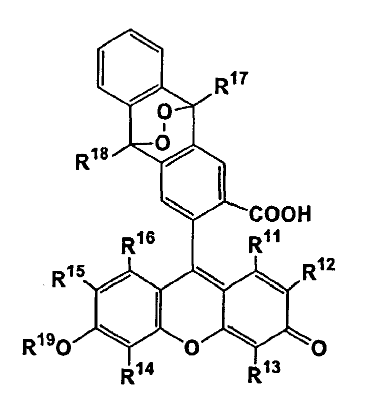 dans laquelle R¹¹, R¹², R¹³, R¹⁴, R¹⁵ et R¹⁶ représentent indépendamment un atome d'hydrogène, un atome d'halogène, un groupe alkyle en C₁ à C₆ ou un groupe alcoxy en C₁ à C₆, R¹⁷ et R¹⁸ représentent indépendamment un groupe alkyle en C₁ à C₄ et R¹⁹ représente un atome d'hydrogène, un groupe alcanoyle en C₁ à C₁₂, ou un groupe acétoxyméthyle, ou un sel de celui-ci.

6. Procédé pour la mesure d'un oxygène singulet, qui comprend les étapes suivantes :
(a) faire réagir un composé représenté par la formule générale (I) ou un sel de celui-ci selon la revendication 1 avec un oxygène singulet, et
(b) mesurer la fluorescence d'un composé représenté par la formule générale (II) ou un sel de celui-ci selon la revendication 5, produit dans l'étape ci-dessus (a).

7. Composé représenté par la formule générale suivante (III) : 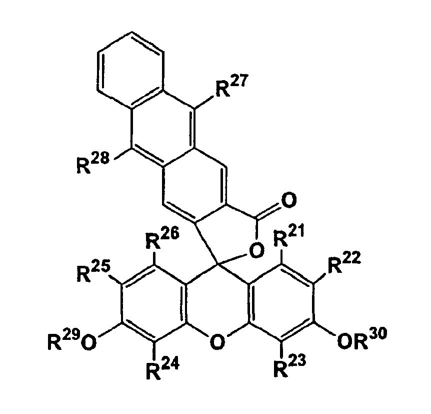 dans laquelle R²¹, R²², R²³, R²⁴ R²⁵ et R²⁶ représentent indépendamment un atome d'hydrogène, un atome d'halogène, un groupe alkyle en C₁ à C₆ ou un groupe alcoxy en C₁ à C₆, R²⁷ et R²⁸ représentent indépendamment un groupe alkyle en C₁ à C₄, et R²⁹ et R³⁰ représentent indépendamment un groupe alcanoyle en C₁ à C₁₂, ou un groupe acétoxyméthyle, ou un sel de celui-ci.

8. Composé ou sel de celui-ci selon la revendication 7, dans lequel R²¹, R²², R²³, R²⁴, R²⁵ et R²⁶ représentent un atome d'hydrogène, R²⁷ et R²⁸ représentent un groupe méthyle, et R²⁹ et R³⁰ représentent un groupe acétyle.

9. Composé ou sel de celui-ci selon la revendication 7, dans lequel R²¹, R²², R²³, R²⁴, R²⁵ et R²⁶ représentent un atome d'hydrogène, R²² et R²⁵ représentent un atome de chlore, R²⁷ et R²⁸ représentent un groupe méthyle et R²⁹ et R³⁰ représentent un groupe acétyle.

10. Agent pour la mesure d'un oxygène singulet, qui comprend un composé ou un sel de celui-ci selon l'une quelconque des revendications 7 à 9.

11. Composé représenté par la formule générale suivante (IV) : 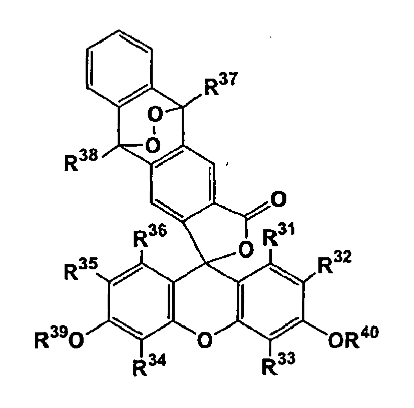 dans laquelle R³¹, R³², R³³, R³⁴, R³⁵ et R³⁶ représentent indépendamment un atome d'hydrogène, un atome d'halogène, un groupe alkyle en C₁ à C₆ ou un groupe alcoxy en C₁ à C₆, R³⁷ et R³⁸ représentent indépendamment un groupe alkyle en C₁ à C₄ et R³⁹ et R⁴⁰ représentent indépendamment un groupe alcanoyle en C₁ à C₁₂, ou un groupe acétoxyméthyle, ou un sel de celui-ci.
